# EUROPÄISCHE PATENTANMELDUNG

(11) **EP 2 607 344 A1**
(43) Veröffentlichungstag der Anmeldung: **26.06.2013**
(21) Anmeldenummer: 11010207.6
(22) Anmeldetag: 24.12.2011
(51) Int. Cl.: C07C 45/62, C07C 45/00

(54) **Verfahren zur Herstellung von cyclischen Ketonen**

(71) Anmelder: AllessaChemie GmbH, 60386 Frankfurt am Main (DE)
(72) Erfinder: Neumann, Doris, Dr., 63069 Offenbach (DE); Ritzer, Joachim, Dr., 63517 Rodenbach (DE); Effenberger, Gunther, Dr., 61118 Bad Vilbel (DE)
(74) Vertreter: Ackermann, Joachim

(57) **Zusammenfassung**

Beschrieben wird ein Verfahren zur Hydrierung von gegebenenfalls substituierten Phenolen an modifizierten Palladium enthaltenden geträgerten Katalysatoren zu Cyclohexanonen. Das gelingt überraschenderweise in ausgewählten alkoholischen Lösungsmitteln mit hoher Selektivität. Dabei gelingt sogar die Rückführung der eingesetzten Katalysatoren, was bisher nur mit erheblichem Verlust an Selektivität möglich war.

## Beschreibung

Die vorliegende Erfindung betrifft ein Verfahren zur Herstellung von cyclischen Ketonen, insbesondere von Cyclohexanonen, durch katalytische Hydrierung von entsprechenden Phenolen.

Es ist bekannt, dass sich Phenole katalytisch zu cyclischen Ketonen hydrieren lassen. So beschreibt US-A-2,829,166 die Hydrierung von Phenolen in Gegenwart von Palladium-Katalysatoren zu den entsprechenden Cyclohexanonen. Aus DE 29 09 780 A1 ist ein Verfahren zur Hydrierung von p-tert.-Amylphenol bekannt. In der EP 731 075 A1 wird ein Verfahren zur Herstellung von substitutierten Cyclohexanonen offenbart. Desweiteren offenbaren EP 889 019 A1 und EP 890 565 A1 1 Verfahren zur Herstellung von Cyclohexanonen durch Hydrierung der entsprechenden Phenole in Gegenwart von Alkanen oder von Wasser als Lösungsmittel.

Gute Selektivitäten an Cyclohexanonen erhält man im Allgemeinen bei Temperaturen von 120 - 250°C und einem Wasserstoffdruck von 1 - 20 bar. Dabei kann es vorteilhaft sein, die Pd/C-Katalysatoren mit einem Alkali- oder Erdalkalimetallcarbonat oder sonstigen basischen oder neutralen Salzen zu behandeln oder dem Hydriergemisch basische bzw. neutrale Salze zuzusetzen.

Bei den zur Phenolhydrierung einsetzbaren Lösungsmitteln ist man allerdings eingeschränkt. Beschrieben ist die Hydrierung in Substanz (vergl. US-A-2,829,166), was aber nur bei Phenolen, die im Bereich der Hydriertemperaturen schmelzen, möglich ist. An weiteren Lösungsmitteln sind lediglich Ether (vergl. EP 731 075 A1), spezielle Alkane wie z.B. Methylcyclohexan (vergl. EP 889 019 A1) oder Ether (vergl. EP 890 565 A1) bekannt. Ferner wird von M. Wydra und H. Vogel in Chemie Ing. Techn. 74, 800-804, 2002, die katalytische Hydrierung von Phenolen in Aromaten als Lösungsmittel, wie z.B. in Toluol und Xylol, beschrieben.

Aus JP 11-060534 A ist ein Verfahren zur Herstellung von Hydroxycyclohexanonen bekannt, bei dem substituierte Polyphenole in Gegenwart eines Alkalimetall enthaltenden Palladiumkatalysators in einem gesättigten einwertigen C₃-C₁₂-Alkohol hydriert werden.

Es ist bekannt, dass beim Einsatz von polareren Lösungsmitteln wie z.B. Alkoholen, die den Anwendungsbereich dieser Reaktion deutlich erweitern würden, wesentlich geringere Umsätze und Selektivitäten auftreten. So beschrieben z.B. Tagaki et al. in Energy & Fuels 13 (6), 1191-1196, 1999, die Kernhydrierung verschiedener Aromaten und fanden, dass im Falle der Katalyse durch Pt/C Alkohole die Reaktivität stark herabsetzen. Higashijima und Nishimura beschrieben in Bull.Chem. Soc. Jpn. 65, 2955-2959, 1992 Untersuchungen zur Kernhydrierung von Phenol und Kresolen an Pd/C-Katalysatoren in verschiedenen Lösungsmitteln und fanden, dass in Alkoholen sowohl die Hydrierrate als auch die Selektivität zu Gunsten der Cyclohexanone, im Vergleich zur Hydrierung in Alkanen oder in Substanz, deutlich herabgesetzt waren. Dieser Effekt wurde beim Einsatz kommerziell erhältlicher Pd/C-Katalysatoren für unterschiedliche primäre, sekundäre und tertitäre Alkohole mit größer gleich 3 Kohlenstoffatomen gefunden; bei sauer behandeltem Pd/C-Katalysator war der Effekt etwas abgeschwächt.

Überraschend wurde nun gefunden, dass sich Phenole in längerkettigen Alkoholen mit hoher Selektivität bei hohem Umsatz zu den entsprechenden cyclischen Ketonen hydrieren lassen, wenn dabei ein ausgewählter Katalysator eingesetzt wird.

Die vorliegende Erfindung betrifft ein Verfahren zur Hydrierung von Phenolen zu cyclischen Ketonen durch Umsetzung der Phenole mit Wasserstoff in Gegenwart eines geträgerten Palladium enthaltenden Katalysators, in einer alkoholischen Lösung, wobei der Alkohol mindestens drei Kohlenstoffatome enthält und der geträgerte Palladium enthaltende Katalysator vor dem Einsatz mit einem neutralen oder basischen Salz, vorzugsweise einem Alkali- und/oder Erdalkali- und/oder Ammoniumsalz behandelt worden ist und/oder die Hydrierung in Gegenwart eines geträgerten Palladium enthaltenden Katalysators und eines neutralen oder basischen Salzes erfolgt.

Bei der Durchführung des erfindungsgemäßen Verfahrens hat sich herausgestellt, dass man gute Selektivitäten an cyclischen Ketonen bei hohem Umsatz der Phenole im Allgemeinen bei Temperaturen von 80 bis 250°C, vorzugsweise von 90 bis 250°C, besonders bevorzugt bei 90 bis 200 °C und ganz besonders bevorzugt bei 100 bis 180°C erhält.

Das erfindungsgemäße Verfahren kann bei unterschiedlichen Wasserstoffdrucken durchgeführt werden. Die Selektivität und der Umsatz der Reaktion sind am höchsten bei einem Wasserstoffdruck von 0,5 - 20 bar. Bevorzugt hydriert man bei 0,5 - 15 bar, besonders bevorzugt bei 1 - 10 bar.

Das erfindungsgemäße Verfahren ist **dadurch gekennzeichnet, dass** man ein Phenol in einem Alkohol mit mindestens drei Kohlenstoffatomen löst, mit einem geträgerten Pd-enthaltenden Katalysator und einem neutralen oder basischen Salz versetzt oder mit einem geträgerten Pd-enthaltenden Katalysator versetzt, der mit einem neutralen oder basischen Salz modifiziert worden ist, und das Phenol bei Reaktionstemperatur gegebenenfalls unter Druck mit Wasserstoff umsetzt. Die Umsetzung erfolgt vorzugsweise in einem Autoklaven.

Als alkoholische Lösungsmittel werden geradkettige oder verzweigte Alkohole mit mindestens drei Kohlenstoffatomen eingesetzt. Dabei handelt es sich um primäre, sekundäre oder tertiäre aliphatische Alkohole mit mindestens drei Kohlenstoffatomen, bevorzugt um geradkettige oder verzweigte sekundäre oder tertiäre aliphatische Alkohole mit mindestens drei Kohlenstoffatomen. Ganz besonders bevorzugt werden sekundäre oder tertiäre Alkohole mit drei bis acht Kohlenstoffatomen verwendet. Beispiele hierfür sind Isopropanol, 2-Butanol, tert-Butanol, 2-Pentanol, 3-Pentanol, 2-Methylbutan-2-ol, 3-Methylbutan-2-ol, Cyclopentanol, Cyclohexanol, 2-Methylpentan-2-ol, 2-Methylpentan-3-ol, 3-Methylpentan-2-ol, 3-Methylpentan-3-ol, 4-Methylpentan-2-ol, 2,3-Dimethyl-2-butanol, 3,3-Dimethyl2-butanol, 2-Hexanol, 3-Hexanol, 2,2-Dimethyl-3-pentanol, 2,3-Dimethyl-3-pentanol, 2,4-Dimethyl-3-pentanol, 4,4-Dimethyl-2-pentanol, 3-Ethyl-3-pentanol, 2-Methyl-2-hexanol, 2-Methyl-3-hexanol, 5-Methyl-2-hexanol, 2-Heptanol, 3-Heptanol, isomere Methylcyclohexanole, 2-Octanol, 3-Octanol, 4-Methylheptan-3-ol, 6-Methylheptan-2-ol, 2-Ethylhexanol und 3,7-Dimethyl-3-octanol.

Die Verdünnung des Phenols im entsprechenden alkoholischen Lösungsmittel wählt man in der Regel zu 10 bis 95 %ig, bevorzugt zu 20 bis 90%ig, besonders bevorzugt zu 25 bis 85%ig.

Neben dem alkoholischen Lösungsmittel kann das erfindungsgemäß eingesetzte Reaktionsgemisch noch weitere mit den geradkettigen oder verzweigten Alkoholen mit mindestens drei Kohlenstoffatomen mischbare Flüssigkeiten aufweisen. So kann beispielsweise ein geringer Anteil an Wasser zugegen sein, beispielsweise bis zu 20 Gew. % an Wasser, vorzugsweise zwischen 0,1 und 10 Gew. % an Wasser, bezogen auf die Gesamtmenge des Lösungsmittels. Die Menge an weiteren mit den Alkoholen mischbaren Flüssigkeiten, insbesondere Wasser, ist im Einzelfall so auszuwählen, dass sich ein einphasiges System ergibt und keine Entmischung erfolgt. Die Anwesenheit von Wasser ist bevorzugt, insbesondere wenn die Hydrierung in Gegenwart eines neutralen oder basischen Salzes vorgenommen wird.

Im erfindungsgemäßen Verfahren kommen geträgerte Palladium enthaltende Katalysatoren zum Einsatz. Als Träger eignen sich eine Vielzahl von Materialien, beispielsweise Aluminiumoxid, keramische Trägermaterialien oder Kohlenstoff bzw. Graphit. Trägermaterialien für diese Katalysatoren sind dem Fachmann bekannt und werden in der Regel in feinverteilter Form verwendet, die gegebenenfalls zu Pellets verpresst sein können. Besonders bevorzugt wird Kohlenstoff, insbesondere Aktivkohle, als Trägermaterial eingesetzt.

Das katalytisch aktive Metall ist Palladium oder eine Kombination von Palladium mit anderen Metallen, beispielsweise mit Platin oder Rhodium.

Der Katalysator kann neben dem katalytisch aktiven Metall noch mit weiteren Komponenten dotiert sein, beispielsweise mit Alkali- oder Erdalkalimetallen.

Ganz besonders wird ein auf Kohlenstoff geträgerter Palladium-Katalysator eingesetzt (nachstehend "Pd/C-Katalysator"). Es können beliebige Kohleträger eingesetzt werden. Diese Pd/C-Katalysatoren sind handelsüblich.

Der geträgerte Katalysator enthält in der Regel von 1 bis 15 Gew. % an katalytisch aktivem Metall, bevorzugt von 1 bis 10 Gew. % und ganz besonders bevorzugt von 1 bis 5 Gew. %. Bevorzugt enthält er 1 - 10% Edelmetall, besonders bevorzugt 1 - 5% Edelmetall. Die geträgerten Katalysatoren können als Feststoffe, z.B. als Pulver oder als Pellets eingesetzt werden oder auch als wasserfeuchte Paste. Der oder die Katalysatoren liegen im Reaktionsgemisch suspendiert vor; die übrigen Bestandteile des Reaktionsgemisches, wie das oder die Lösungsmittel, das Phenol und das hydrierte Phenol liegen in der Regel als Einphasengemisch vor bilden eine Lösung, die mit dem in der Gasphase vorliegenden Wasserstoff reagiert.

Bezogen auf das Phenol werden diese Katalysatoren in Mengen von 1 x 10⁻⁴ bis 1 Gew.-% (gerechnet jeweils als Pd-Metall) eingesetzt. Bevorzugt werden sie in Mengen von 1 x 10⁻³ bis 1 Gew.-% eingesetzt, besonders bevorzugt in Mengen von 1 x 10⁻³ bis 0,1 Gew.-%.

Der im erfindungsgemäßen Verfahren eingesetzte Katalysator wird vor dem Einsatz mit einem neutralen oder basischen Salz, in der Regel mit einer Lösung eines neutralen oder basischen Alkali-, Erdalkali- oder Ammoniumsalzes behandelt oder das neutrale oder basische Salz wird dem Hydriergemisch zugesetzt. In letzterem Fall ist es möglich auch vorher unbehandelte geträgerte Katalysatoren einzusetzen. Das neutrale oder basische Salz kann als Substanz dem geträgerten Katalysator zugesetzt werden oder vorzugsweise als Lösung in einem geeigneten Lösungsmittel, insbesondere als wässrige Lösung. Als neutrale oder basische Salze setzt man Metallsalze ein, bevorzugt Hydroxide, Carbonate, Hydrogencarbonate, Phosphate, Monohydrogenphosphate, Acetate oder Borate von Alkalimetallen, wie von Lithium, Natrium, Kalium, Rubidium oder Cäsium; oder von Erdalkalimetallen, wie von Beryllium, Magnesium, Calcium, Strontium oder Barium; oder von Ammonium. Besonders bevorzugt werden Hydroxide, Carbonate, Hydrogencarbonate, Phosphate, Monohydrogenphosphate, Acetate oder Borate von Lithium, Natrium, Kalium, Magnesium, Calcium, Strontium oder Ammonium eingesetzt. Beispiele sind Lithium-, Natrium-, Kalium- oder Ammoniumhydroxid, Natrium-, Kalium- oder Ammoniumhydrogencarbonat, Lithium-, Natrium-, Kalium-, Ammonium-, Magnesium- oder Calciumcarbonat, Lithium-, Natrium-, Kalium-, Ammonium- oder Magnesiumphosphat, Lithium-, Natrium-, Kalium-, Ammonium- oder Calciumacetat, oder Lithium-, Natrium-, Kalium- oder Ammoniumborat. Es können auch Gemische von neutralen oder basischen Salzen eingesetzt werden.

Unter einem neutralen oder basischen Salz ist im Rahmen dieser Erfindung ein Salz zu verstehen, das als 0,1 - 15%ige wässrige Lösung einen pH-Wert von 7 oder von größer als 7 aufweist.

Dieses dem Katalysator und/oder der Reaktionslösung zugesetzte neutrale oder basische Salz gibt man in Mengen von 0,001 bis 5 Mol-% bezogen auf eingesetztes Phenol zu. Bevorzugt gibt man das Salz in Mengen von 0,005 bis 5 Mol-% zu, besonders bevorzugt in Mengen von 0,005 bis 1 Mol-%. Die Behandlung oder Zugabe kann mit dem Salz in Lösung erfolgen, beispielsweise in Wasser oder in alkoholischen Lösungsmitteln.

Vorzugsweise setzt man das neutrale oder basische Salz als wässrige Lösung dem geträgerten Katalysator oder der Reaktionsmischung zu. Das Salz wird zur Herstellung der wässrigen Lösung im allgemeinen in der 1 bis 1000-fachen Menge an Wasser, bezogen auf das Salz, aufgelöst. Der Anteil an Wasser sollte so gewählt werden, dass das Wasser mit dem Alkohol mit mindestens drei Kohlenstoffatomen eine homogene Mischung und kein Mehrphasensystem bildet.

Der im erfindungsgemäßen Verfahren eingesetzte geträgerte Palladium-Katalysator kann frisch sein oder es kann ein bereits ein- oder mehrmals verwendeter Katalysator eingesetzt werden. Es hat sich überraschenderweise gezeigt, dass sogar die Rückführung der eingesetzten Katalysatoren möglich ist, was bei nicht auf diese Weise behandelten Katalysatoren bzw. bei Reaktionsführung in anderen Lösungsmitteln nur mit erheblichem Verlust an Selektivität möglich war.

Als Phenole können im erfindungsgemäßen Verfahren beliebige Verbindungen eingesetzt werden, welche mindestens einen aromatischen Kern und damit kovalent verbunden mindestens eine Hydroxylgruppe aufweisen.

Bei den Phenolen kann es sich um ein- oder mehrkernige carbocyclische aromatische Verbindungen handeln, vorzugsweise um drei-, zwei- oder insbesondere einkerninge carbocyclische aromatische Verbindungen; oder es kann sich um ein- oder mehrkernige heterocyclische aromatische Verbindungen handeln, die vorzugsweise ein oder zwei Ringheteroatome ausgewählt aus der Gruppe Stickstoff, Sauerstoff und Schwefel oder Kombinationen davon aufweisen.

Diese carbocyclischen oder heterocyclischen aromatischen Verbindungen können ein oder mehrere phenolische Hydroxylgruppe aufweisen, und gegebenenfalls einen oder mehrere Substituenten, beispielsweise Substituten ausgewählt aus der Gruppe der Alkyl-, Alkoxy-, Hydroxyalkyl-, Cycloalkyl-, Cycloalkoxy-, Aryl-, Aryloxy-, Aralkyl-, Aralkyloxy-, Carbonsäure-, Sulfonsäure-, Amino-, Nitro-, Carbonsäureester-, Carbonsäureamid-, Sulfonsäureester-, Sulfonsäureamid- und/oder Nitrilgruppen oder von Kombinationen von zwei oder mehreren dieser Gruppen bzw. Atome.

Vorzugsweise werden carbocyclische oder heterocyclische aromatische Verbindungen mit nur einer phenolischen Hydroxylgruppe eingesetzt.

Beispiele für Alkylgruppen sind geradkettige oder verzweigte Alkylgruppen mit ein bis zehn Kohlenstoffatomen, wie Methyl, Ethyl, Propyl, Isopropyl, n-Butyl, sek.-Butyl, tert-Butyl, Pentyl, n-Hexyl, n-Heptyl, 2-Ethylhexyl, n-Octyl, n-Nonyl oder n-Decyl.

Beispiele für Alkoxygruppen sind solche mit geradkettigen oder verzweigten Alkylgruppen mit ein bis zehn Kohlenstoffatomen, wie Methoxy, Ethoxy, Isopropoxy, n-Butoxy, sek.-Butoxy, tert-Butoxy, Pentyloxy, n-Hexyloxy, n-Heptyloxy, 2-Ethylhexyloxy, n-Octyloxy, n-Nonyloxy oder n-Decyloxy.

Beispiele für Cycloalkylgruppen sind solche mit fünf oder sechs Ringkohlenstoffatomen, die ihrerseits wiederum substitutiert sein können, beispielsweise mit Alkylgruppen. Ein Beispiel für eine Cycloalkylgruppe ist Cyclohexyl.

Beispiele für Cycloalkoxygruppen sind solche mit fünf oder sechs Ringkohlenstoffatomen im Cycloalkylring, der seinerseits wiederum substitutiert sein kann, beispielsweise mit Alkylgruppen. Ein Beispiel für eine Cycloalkoxygruppe ist Cyclohexyloxy.

Beispiele für Arylgruppen sind solche mit sechs oder zehn Ringkohlenstoffatomen im Arylring, die ihrerseits wiederum substitutiert sein können, beispielsweise mit Alkylgruppen. Ein Beispiel für eine Arylgruppe ist Phenyl.

Ein Beispiel für eine Aralkylgruppe ist Benzyl, das seinerseits wiederum substitutiert sein kann, beispielsweise mit Alkylgruppen.

Ein Beispiel für eine Carbonsäureamidgruppe ist C₁-C₄-Acylamino, vorzugsweise Acetylamino.

Die carbocyclischen oder heterocyclischen aromatischen Verbindungen können neben aromatischen Ringen noch nicht-aromatische gesättigte oder ethylenisch ungesättigte Ringe aufweisen, welche mit den aromatischen Ringen annelliert sind oder mit den aromatischen Ringen über kovalente Bindungen verknüpft sind oder mit den aromatischen Ringen ein bi- oder polycyclisches System bilden.

Beispiele für besonders bevorzugt eingesetzte Phenole sind solche, die sich von Phenylgruppen, substitutierten Phenylgruppen, Naphthylgruppen, substituierten Naphthylgruppen, Anthracenylgruppen und substitutierten Anthracenylgruppen ableiten und die vorzugsweise eine phenolische Hydroxylgruppen aufweisen.

Besonders bevorzugt werden Phenole der Formel (I) eingesetzt 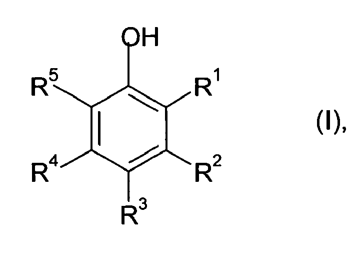 worin R¹, R², R³, R⁴, R⁵ unabhängig voneinander Wasserstoff, C₁-C₁₀-Alkyl, C₃-C₆-Cycloalkyl, C₆-C₁₀-Aryl, C₃-C₁₀-Heteroaryl mit O, N und/oder S als RingHeteroatome, C₆-C₁₀-Aryl-CH₂, C₆-C₁₀-Aryl-O, C₁-C₁₀-Alkoxy, C₃-C₈-Cycloalkoxy, N-C₁-C₄-Alkylamino, N,N-Di-C₁-C₄-alkylamino, C₁-C₄-Acylamino, COOR⁶ mit R⁶ = Wasserstoff, C₁-C₄-Alkyl oder -CH₂-R⁷ mit R⁷ = Hydroxyl, C₁-C₄-Alkyoxy, N-C₁-C₄-Alkylamino oder N, N-Di-C₁-C₄-alkylamino bedeuten.

Diese Phenole werden durch das erfindungsgemäße Verfahren in hoher Selektivität zu den entsprechenden Cyclohexanonen der allgemeinen Formel (II) hydriert 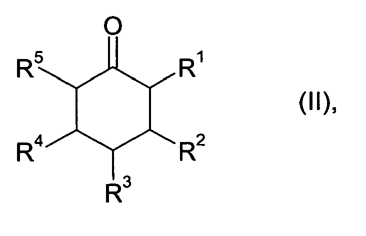 wobei R¹, R², R³, R⁴, R⁵ die oben definierte Bedeutung haben.

In den Formeln (I) und (II) stehen R¹, R², R³, R⁴, R⁵ unabhängig voneinander bevorzugt für Wasserstoff, C₁-C₆-Alkyl, C₁-C₆-Alkoxy oder C₁-C₄-Acylamino, wobei bevorzugt 1 bis 4 der Reste R¹ bis R⁵ von Wasserstoff verschieden sind.

Besonders bevorzugt stehen R¹, R², R⁴ und R⁵ für Wasserstoff und R³ für C₁-C₁₀ Alkyl, C₁-C₁₀-Alkoxy oder C₁-C₄-Acylamino.

Das beschriebene Verfahren kann in beliebigen Reaktoren durchgeführt werden. Bevorzugte Beispiele dafür sind Rühr- oder Schlaufenreaktoren.

Die Hydrierzeiten des beschriebenen Verfahrens liegen im Allgemeinen zwischen ein und drei Stunden. Im Einzelfall können aber auch kürzere oder längere Reaktionszeiten verwendet werden.

Die erreichten Selektivitäten an cyclischen Ketonen nach dem erfindungsgemäßen Verfahren liegen häufig oberhalb von 94%, teils bei bis zu 96%.

Die Vorteile des erfindungsgemäßen Verfahrens liegen darin, dass die Palette an einsetzbaren Lösungsmitteln deutlich erweitert werden konnte. Damit besteht die Möglichkeit, bisher auf Grund schlechter Löslichkeiten oder hoher Schmelzpunkte nicht einsetzbare Phenole, zu cyclischen Ketonen in Lösung zu hydrieren. Ein weiterer großer Vorteil ist, dass die in dem beschriebenen Verfahren eingesetzten Katalysatoren mehrfach wieder eingesetzt werden können, ohne dass es zu einem Verlust an Selektivität kommt. Dies reduziert den Edelmetallverbrauch stark und stellt somit einen enormen Kostenvorteil dar.

Als noch weiterer großer Vorteil ist die Durchführbarkeit des erfindungsgemäßen Verfahrens in der Gegenwart von Wasser ansehen. Durch das Entfallen von aufwendigen Trocknungsmaßnahmen wird die Produktivität des Verfahrens erheblich gesteigert.

Die nachfolgenden Beispiele illustrieren die Erfindung ohne diese einzuschränken.

### Beispiele

Die Versuche wurden mit handelsüblichen alkalischen Pd/C-Katalysatoren (3% Pd/C, ca. 50% wasserfeucht) durchgeführt. Die eingesetzten Rohstoffe und Lösungsmittel stammten aus dem Chemikalienhandel.

### Beispiel 1: Herstellung von 4-tert. Butylcyclohexanon

250 g 4-tert-Butylphenol wurden gelöst in 135 g tert.Butanol und dazu wurden 1,8 g eines mit Natriumcarbonat, Lithiumcarbonat oder Natrium- oder Lithiumacetat versetzten Pd/C-Katalysators und 2,4 g Wasser zugesetzt. Man hydrierte bei 160°C/6 bar Wasserstoffdruck. Die Reaktion war innerhalb 2 Stunden abgeschlossen. Die Reaktionslösungen enthielten 89% 4-tert-Butylcyclohexanon.

### Beispiel 2: Herstellung von 4-tert. Butylcyclohexanon

250 g 4-tert-Butylphenol wurden gelöst in 135 g tert.Butanol und dazu wurden 1,8 g eines mit Kaliumcarbonat oder Cäsiumcarbonat versetzten Pd/C-Katalysators zugesetzt. Man hydrierte bei 160°C/6 bar Wasserstoffdruck. Die Reaktion war innerhalb 2 Stunden abgeschlossen. Die Reaktionslösungen enthielten 87% 4-tert-Butylcyclohexanon.

### Beispiel 3: Herstellung von 4-tert. Butylcyclohexanon

250 g 4-tert-Butylphenol wurden gelöst in 135 g tert.Amylalkohol und dazu wurden 1,8 g eines mit Natriumcarbonat, Lithiumcarbonat oder Lithium- oder Natriumacetat versetzten Pd/C-Katalysators zugesetzt. Man hydrierte bei 160°C/6 bar Wasserstoffdruck. Die Reaktion war innerhalb 2 Stunden abgeschlossen. Die Reaktionslösungen enthielten 87% 4-tert-Butylcyclohexanon.

### Beispiel 4: Herstellung von 4-tert. Butylcyclohexanon

250 g 4-tert-Butylphenol wurden gelöst in 135 g 2-Butanol und dazu wurden 1,8 g eines mit Natriumcarbonat, Lithiumcarbonat oder Lithium- oder Natriumacetat versetzten Pd/C-Katalysators und 2,4 g Wasser zugesetzt. Man hydrierte bei 160°C/6 bar Wasserstoffdruck. Die Reaktion war innerhalb 2 Stunden abgeschlossen. Die Reaktionslösungen enthielten 91% 4-tert-Butylcyclohexanon.

### Beispiel 5: Herstellung von 4-tert. Butylcyclohexanon

300 g 4-tert-Butylphenol wurden gelöst in 125 g 4-Methylpentan-2-ol und dazu wurden 2,9 g eines mit Natriumcarbonat, Lithiumcarbonat oder Natrium- oder Lithiumacetat versetzten Pd/C-Katalysators zugesetzt. Man hydrierte bei 160°C/6 bar Wasserstoffdruck. Die Reaktion war innerhalb 1 Stunde abgeschlossen. Die Reaktionslösungen enthielten 94 bis 96% 4-tert-Butylcyclohexanon.

### Beispiel 6: Herstellung von 4-Methoxycyclohexanon

250 g 4-Methoxyphenol wurden in 125 g tert-Butanol bei 150°C/5 bar Wasserstoffdruck an 2,9 g eines Pd/C-Katalysators, der mit Natrium- oder Lithiumcarbonat oder Natrium- oder Lithiumacetat versetzt worden war, innerhalb 1,7 h hydriert. Die Hydrierlösung enthielt 93% 4-Methoxycyclohexanon.

### Beispiel 7: Herstellung von 4-Methoxycyclohexanon

250 g 4-Methoxyphenol wurden in 125 g 4-Methylpentan-2-ol bei 140°C/4 bar Wasserstoffdruck innerhalb 3 h an einem mit Lithium- oder Natriumcarbonat oder Lithium- oder Natriumacetat versetzten Pd/C-Katalysators hydriert. In der Hydrierlösung lagen 92 bis 94% 4-Methoxycyclohexanon vor.

### Beispiel 8: Herstellung von 4-Methylcyclohexanon

250 g p-Kresol wurden in 130 g 2-Butanol gelöst und bei 170°C/6 bar Wasserstoffdruck an 3,2 g eines mit Lithium- oder Natriumcarbonat oder Lithium- oder Natriumacetat versetzten Pd/C-Katalysators hydriert. Die Hydrierzeit lag bei 3 h. Die Reaktionslösung enthielt 93% 4-Methylcyclohexanon.

### Beispiel 9: Herstellung von 3-Methylcyclohexanon

250 g m-Kresol wurden in 144 g 4-Methylpentan-2-ol gelöst und bei 170°C/6 bar Wasserstoffdruck an 3,3 g eines mit Natrium- oder Lithiumcarbonat oder Natrium- oder Lithiumacetat versetzten Pd/C-Katalysators innerhalb von 1,5 h hydriert. Das Reaktionsgemisch enthielt 96% 3-Methylcyclohexanon.

### Beispiel 10: Herstellung von 2-Methylcyclohexanon

250 g o-Kresol wurden in 144 g 4-Methylcyclohexanon gelöst und bei 170°C/6 bar Wasserstoffdruck an 3,3 g eines mit Natrium- oder Lithiumcarbonat oder Natrium- oder Lithiumacetat versetzten Pd/C-Katalysators hydriert. Die Hydrierzeit lag bei 3,5 h. In der Reaktionslösung detektierte man 95% 2-Methylcyclohexanon.

### Beispiel 11: Herstellung von 4-Methylcyclohexanon

Man löste 250 g p-Kresol in 142 g Pentan-2-ol und hydrierte bei 160°C/6 bar Wasserstoffdruck an 3,3 g eines mit Natrium- oder Lithiumcarbonat oder Natrium- oder Lithiumacetat versetzten Pd/C-Katalysators. Die Reaktionszeit betrug 2,5 h, in der Reaktionslösung lagen 94% 4-Methylcyclohexanon vor.

### Beispiel 12: Herstellung von 4-Methylcyclohexanon

250 g p-Kresol wurden in 144 g Cyclohexanol gelöst und bei 160°C/6 bar Wasserstoffdruck an 3,3 g eines mit Natrium- oder Lithiumcarbonat oder Natrium- oder Lithiumacetat versetzten Pd/C-Katalysators hydriert. Nach einer Hydrierzeit von 3 h enthielt das Reaktionsgemisch 91% 4-Methylcyclohexanon.

### Beispiel 13: Herstellung von 4-Methylcyclohexanon

250 g 4-Methoxyphenol wurden gelöst in 125 g 4-Methylpentan-2-ol und bei 140°C/3,5 bar Wasserstoffdruck an 3,7 g eines recyclisierten Pd/C-Katalysators, der nur vor seinem Ersteinsatz mit Natrium- oder Lithiumcarbonat oder Natrium- oder Lithiumacetat versetzt worden war, hydriert. Nach einer Hydrierzeit von 4,7 h lagen in der Reaktionslösung 92% 4-Methoxycyclohexanon vor.

### Beispiel 14: Herstellung von 4-tert.Butylcyclohexanon

300 g 4-tert-Butylphenol wurden in 125 g 4-Methylpentan-2-ol gelöst und bei 160°C/6 bar Wasserstoffdruck an 4,0 g eines recyclisierten Pd/C-Katalysators, der nur vor seinem Ersteinsatz mit Natrium- oder Lithiumcarbonat oder Natrium- oder Lithiumacetat versetzt worden war, hydriert. Die Hydrierzeit lag bei 3,5 h. Das Reaktionsgemisch enthielt 94% 4-tert-Butylcyclohexanon.

### Beispiel 15: Herstellung von 4-tert.Butylcyclohexanon

Man löste 300 g 4-tert-Butylphenol in 125 g 4-Methylpentan-2-ol und hydrierte bei 160°C/6 bar Wasserstoffdruck an 2,9 g eines mit Natriumtetraborat vorbehandelten Pd/C-Katalysators. Die Hydrierzeit betrug 50 min, im Reaktionsgemisch lagen 89% 4-tert-Butylcyclohexanon vor.

### Beispiel 16: Herstellung von 4-tert.Butylcyclohexanon

300 g tert-Butylphenol wurden in 125 g 4-Methylpentan-2-ol gelöst und bei 160°C/6 bar Wasserstoffdruck an 1,7 g eines mit basischem Magnesiumcarbonat und Natriumcarbonat versetzten Pd/C-Katalysators hydriert. Die Hydrierzeit lag bei 1,3 h. Das Reaktionsgemisch enthielt 93% 4-tert-Butylcyclohexanon.

### Vergleichsbeispiele:

### Beispiel 17: Hydrierung an einem nicht-alkalisierten Katalysator

250 g 4-Methoxyphenol wurden gelöst in 125 g 4-Methylpentan-2-ol und bei 140°C/3,5 bar Wasserstoffdruck an 2,9 g eines nicht vorher mit einem basischen oder neutralen Salz versetzten Pd/C-Katalysators hydriert. Nach einer Hydrierzeit von 5 h lagen in der Reaktionslösung 77% 4-Methoxycyclohexanon vor.

### Beispiel 18: Hydrierung in Isooctan

20 kg tert-Butylphenol, gelöst in 20 kg Isooctan wurden an 0,071 g eines mit Natriumcarbonat alkalisierten getrockneten Pd/C-Katalysators bei 150°C/5 bar Wasserstoffdruck innerhalb 3 h hydriert. Im Reaktionsgemisch wurden 80% 4-tert-Butylcyclohexanon detektiert.

Setzte man den Katalysator 50% wasserfeucht, d.h. in der handelsüblichen Form ein, so verklumpte er. Es fand keine Wasserstoffaufnahme statt.

## Patentansprüche

1. Verfahren zur Hydrierung von Phenolen zu cyclischen Ketonen durch Umsetzung der Phenole mit Wasserstoff in Gegenwart eines Katalysators in einer Lösung enthaltend einen Alkohol mit mindestens drei Kohlenstoffatomen, wobei ein geträgerter Palladium enthaltender Katalysator verwendet wird, der vor dem Einsatz mit einem neutralen oder basischen Salz behandelt worden ist und/oder wobei die Hydrierung in Gegenwart eines geträgerten Palladium enthaltenden Katalysators und eines neutralen oder basischen Salzes erfolgt.

2. Verfahren nach Anspruch 1, **dadurch gekennzeichnet, dass** die Umsetzung bei Temperaturen von 80 bis 250°C, vorzugsweise von 120 bis 250°C, besonders bevorzugt bei 90 bis 200 °C und ganz besonders bevorzugt bei 100 bis 180°C erfolgt.

3. Verfahren nach Anspruch 1, **dadurch gekennzeichnet, dass** die Umsetzung bei einem Wasserstoffdruck von 0,5 bis 20 bar, bevorzugt von 0,5 bis 15 bar, und besonders bevorzugt bei 1 bis 10 bar erfolgt.

4. Verfahren nach Anspruch 1, **dadurch gekennzeichnet, dass** als Alkohol mit mindestens drei Kohlenstoffatomen ein geradkettiger oder verzweigter sekundärer oder tertiärer aliphatischer Alkohol mit mindestens drei Kohlenstoffatomen, besonders bevorzugt ein sekundärer oder tertiärer Alkohol mit drei bis acht Kohlenstoffatomen eingesetzt wird, ganz besonders bevorzugt ein Alkohol aus der Gruppe Isopropanol, 2-Butanol, tert-Butanol, 2-Pentanol, 3-Pentanol, 2-Methylbutan-2-ol, 3-Methylbutan-2-ol, Cyclopentanol, Cyclohexanol, 2-Methylpentan-2-ol, 2-Methylpentan-3-ol, 3-Methylpentan-2-ol, 3-Methylpentan-3-ol, 4-Methylpentan-2-ol, 2,3-Dimethyl-2-butanol, 3,3-Dimethyl2-butanol, 2-Hexanol, 3-Hexanol, 2,2-Dimethyl-3-pentanol, 2,3-Dimethyl-3-pentanol, 2,4-Dimethyl-3-pentanol, 4,4-Dimethyl-2-pentanol, 3-Ethyl-3-pentanol, 2-Methyl-2-hexanol, 2-Methyl-3-hexanol, 5-Methyl-2-hexanol, 2-Heptanol, 3-Heptanol, 2-Octanol, 3-Octanol, 4-Methylheptan-3-ol, 6-Methylheptan-2-ol, 2-Ethylhexanol oder 3,7-Dimethyl-3-octanol.

5. Verfahren nach Anspruch 1, **dadurch gekennzeichnet, dass** die Umsetzung in einer wässrigen alkoholischen Lösung stattfindet, deren Wassergehalt bis zu 20 Gew. %, bezogen auf die Menge an Gesamtlösungmittel, beträgt.

6. Verfahren nach Anspruch 1, **dadurch gekennzeichnet, dass** die Konzentration des Phenols im Alkohol 10 bis 95 Gew. % beträgt.

7. Verfahren nach Anspruch 1, **dadurch gekennzeichnet, dass** als geträgerter Palladium enthaltender Katalysator ein auf Kohlenstoff geträgerter Palladium-Katalysator eingesetzt wird.

8. Verfahren nach Anspruch 1, **dadurch gekennzeichnet, dass** der geträgerte Palladium enthaltende Katalysator 1 bis 15 Gew. %, bevorzugt 1 bis 10 Gew. % und ganz besonders bevorzugt von 1 bis 5 Gew. % an katalytisch aktivem Metall enthält.

9. Verfahren nach Anspruch 1, **dadurch gekennzeichnet, dass** der geträgerte Palladium enthaltende Katalysator vor dem Einsatz mit einem neutralen oder basischen Salz, vorzugsweise mit einer neutralen oder basischen wässrigen Lösung eines Alkali-, Erdalkali- oder Ammoniumsalzes behandelt wird, ganz besonders bevorzugt mit einer neutralen oder basischen wässrigen Lösung, welche die 1- bis 100-fache Menge an Wasser, bezogen auf das Salz, enthält.

10. Verfahren nach Anspruch 9, **dadurch gekennzeichnet, dass** das neutrale oder basische Salz ein Hydroxid, Carbonat, Hydrogencarbonat, Phosphat, Monohydrogenphosphat, Acetat oder Borat von Lithium, Natrium, Kalium, Rubidium, Cäsium, Beryllium, Magnesium, Calcium, Strontium, Barium oder Ammonium ist, ganz besonders bevorzugt Lithium-, Natrium-, Kalium- oder Ammoniumhydroxid, Natrium-, Kalium- oder Ammoniumhydrogencarbonat, Lithium-, Natrium-, Kalium-, Ammonium- Magnesium oder Calciumcarbonat,
Lithium-, Natrium-, Kalium, Ammonium- oder Magnesiumphosphat, Lithium-, Natrium-, Kalium-, Ammonium- oder Calciumacetat oder Lithium-, Natrium-, Kalium- oder Ammoniumborat ist.

11. Verfahren nach Anspruch 1, **dadurch gekennzeichnet, dass** der geträgerte Palladium-Katalysator, bezogen auf das eingesetzte Phenol, in Mengen von 1 x 10⁻⁴ bis 1 Gew.-% (gerechnet jeweils als Pd-Metall) eingesetzt wird.

12. Verfahren nach Anspruch 1, **dadurch gekennzeichnet, dass** der eingesetzte geträgerte Palladium-Katalysator frisch ist oder bereits ein- oder mehrmals verwendet worden ist.

13. Verfahren nach Anspruch 1, **dadurch gekennzeichnet, dass** man das dem Katalysator zugesetzte neutrale oder basische Salz in Mengen von 0,001 - 5 Mol-%, bezogen auf eingesetztes Phenol, zugibt.

14. Verfahren nach Anspruch 1, **dadurch gekennzeichnet, dass** als Phenol eine
Verbindung der Formel (I) eingesetzt wird 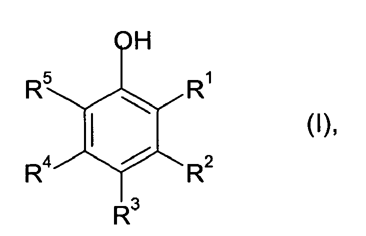 worin R¹, R², R³, R⁴, R⁵ unabhängig voneinander Wasserstoff, C₁-C₁₀-Alkyl, C₃-C₆-Cycloalkyl, C₆-C₁₀-Aryl, C₃-C₁₀-Heteroaryl mit O, N und/oder S als RingHeteroatome, C₆-C₁₀-Ary)-CH₂, C₆-C₁₀-Aryl-O, C₁-C₁₀-Alkoxy, C₃-C₈-Cycloalkoxy, N-C₁-C₄-Alkylamino, N,N-Di-C₁-C₄-alkylamino, C₁-C₄-Acylamino, COOR⁶ mit R⁶ = Wasserstoff, C₁-C₄-Alkyl oder -CH₂-R⁷ mit R⁷ = Hydroxyl, C₁-C₄-Alkyoxy, N-C₁-C₄-Alkylamino oder N, N-Di-C₁-C₄-alkylamino bedeuten, worin R¹, R², R³, R⁴ und R⁵ vorzugsweise unabhängig voneinander für Wasserstoff, C₁-C₁₀-Alkyl, C₁-C₁₀-Alkoxy oder C₁-C₄-Alkylamino stehen und ein bis vier dieser Reste von Wasserstoff verschieden sind, und worin ganz besonders bevorzugt R¹, R², R⁴ und R⁵ unabhängig voneinander für Wasserstoff, C₁-C₁₀-Alkyl, C₁-C₁₀-Alkoxy oder C₁-C₄-Alkylamino stehen.
